# EUROPEAN PATENT APPLICATION

(11) **EP 1 672 080 A1**
(43) Date of publication of application: **21.06.2006**
(21) Application number: 04292994.3
(22) Date of filing: 14.12.2004
(51) Int. Cl.: C12Q 1/68

(54) **Method for universal PCR**

(71) Applicant: INSTITUT NATIONAL DE LA SANTE ET DE LA RECHERCHE MEDICALE (INSERM), 75654 Paris Cédex 13 (FR); INSTITUT PASTEUR, 75724 Paris Cédex 15 (FR)
(72) Inventor: Pineau, Pascal, 92170 Vanves (FR); Vartanian, Jean-Pierre, 75004 Paris (FR); Dejean, Anne, 75014 Paris (FR); Wain-Hobson, Simon, 78180 Montigny le Bretonneux (FR)
(74) Representative: Tanty, François

(57) **Abstract**

The instant invention relates to a method for amplifying a genomic DNA sequence, comprising at least steps of:
i) contacting said genomic DNA sequence with a set of pairs of degenerate oligonucleotide primers hybridizing with primers binding sites of said genomic DNA sequence, each of these sites being homologously present in at least two orthologous forms of said genomic DNA sequence,
ii) subjecting the mixture obtained at step i) to a touchdown polymerase chain reaction (PCR) under suitable conditions for obtaining said amplified DNA sequence.

## Description

The instant invention relates to oligonucleotide primers useful for a PCR, a method for amplifying a genomic DNA sequence using those oligonucleotide primers, a method for identifying species belonging to superior eukaryotes comprising the use of said method, and a kit comprising oligonucleotide primers according to the invention.

More particularly, the instant invention relates to a method for amplifying a genomic DNA sequence comprising a touchdown PCR, said PCR being carried out with a set of pairs of degenerate oligonucleotide primers.

The construction of evolutionary trees or the identification of a species by DNA analysis requires the possibility of disposing of a sufficient amount of genetic material. The harvesting of sufficient amount of genetic material is, typically, performed by amplifying the available nucleic acid sequences by polymerase chain reaction.

The polymerase chain reaction (PCR) permits the cloning and analysis of DNA. This method allows for selectively amplifying a specific target DNA sequence within a heterogeneous collection of DNA sequences, such as total genomic DNA or complex cDNA population.

To perform such a selective amplification, some prior DNA sequence information from the target sequences is required. This information is used to design two oligonucleotide primers which are specific for the target sequence, and the length of which is, typically, from about 15 to about 30 mers. The primers bind specifically to complementary DNA sequences at the target site, and in the presence of a suitably heat-stable DNA polymerase and DNA precursors (the four deoxynucleoside triphosphate, dATP, dCTP, dGTP and dTTP), they initiate the synthesis of new DNA strings which are complementary to the individual DNA string of the target DNA sequence.

However, it is not always possible to dispose of the required information regarding the target DNA sequence to be amplified for selecting or designing the oligonucleotide primers.

Since the appearance of PCR, several tests for species identification or evolutionary tree construction have been described, which enable DNA amplification from most vertebrate species without true knowledge of the DNA sequence to be amplified. However, these assays are directed to conserved sequences of the mitochondrial genome or at ribosomal gene, both present at several hundred to several thousand copies per cell in metazoan organism. Such targets are readily detectable but reflect only distantly the status of non ribosomal nuclear agents in tissues (Bataille *et al.,* Forensic Sci. Int., 1999, 99:165-170; Meyer et al., J. AOAC Int., 1994, 77:617-622; Naito *et al.,* J. Forensic Sci., 1992, 37:396-403). In addition, mitochondrial DNA regularly migrates to chromosomal DNA where fragments may be stabilized as pseudogenes (Bensasson *et al.,* Mol. Biol. Evol., 2001, 18:246-53; Wallace *et al.,* Proc. Natl. Acad. Sci. USA, 1997, 94 :14900-5).

Consequently, mistaking nuclear mitochondrial DNA for mitochondrial genomes may lead to erroneous conclusions when performing species identification (Vartanian and Wain-Hobson, Proc. Natl. Acad. Sci. USA, 2002, 99:7566-7569).

In the forensic field, molecular biology tools may be applied in order to identify biological samples taken from criminal scenes. However, such biological samples often contain very ancient or degraded DNA. In addition, the amount of DNA available from these highly degraded biological sample may fall sometimes in the range of femtogram values, and requires very high sensitive method to be detected.

The forensic entomology is related to the characterization of larvae of dipters taken from corpses in order to assess the post-mortem interval. Typically, after having removed larvae from the corpse, a period of culture is required to allow the larvae to be transformed in adult form of the dipters. Therefore, a correct identification of the insects colonizing the corpse may take some time that may range from a few days to some weeks, according to the species of the dipters. Such required period of time results in the delaying of the identification and thus of the police investigation.

The characterization of the larvae by amplification of mitochondrial DNA gene sequences coding for the subunit I and II of the cytochrome oxydase B (CO I and CO II), allows to bypass the culture steps and to accelerate the process of identification of the larvae. However, as already above-discussed, such a method, based on DNA mitochondrial analysis, may lead to some misinterpretations.

Therefore, there is a need for a molecular analysis tool allowing for a simple and accurate identification of various animal species.

There is also a need for a molecular analysis tool allowing the study of genomic DNA from phylogenetically very distant animals.

There is also a need for a molecular tool allowing an accurate quality control of the genomic DNA from samples from different animal species.

There is also a need for a molecular analysis tool which is cost effective and whose specificity is robust.

There is also a need for a highly sensitive molecular analysis tool allowing detection of DNA and identification of a species by starting from a tiny amount of DNA.

There is also a need for a molecular analysis tool which is versatile and may be used in forensic investigation, as well as in quality control of samples, or for establishing accurate evolutionary trees.

The instant invention aims, notably, to satisfy the overall of those needs as well as avoiding the drawbacks previously discussed.

The inventors have identified sets of pairs of degenerate oligonucleotide primers usable in a method for amplifying a genomic DNA sequence. This genomic DNA sequence may be all or part of a gene encoding for a protein, the structure of which being very conserved among species. More precisely, this protein may belong to the histones family which are functional and structural components of chromatin.

Five classes of histones were originally characterized (H1, H2A, H2B, H3 and H4). In mammals, the gene family coding for histone H4 comprises a few number of nuclear genes (14 genes in human genome). They are considered as some of the most conserved genes in eukaryote (DeLange and Smith, Annu. Rev. Biochem., 1971, 40:279-314; Thatcher and Gorovsky, Nucleic Acid Res., 1994, 22:174-179; Wells and Brown, Nucleic Acid Res., 1991, 19:2173-88). Each of the 14 human histone H4 genes encodes the same protein.

The inventors have observed that a genomic DNA sequence of all or part of a gene of the histone H4 family, notably the gene HIST2H4, may be used as an appropriate target for use in a method for quality control of DNA as well as for identification of superior eukaryote species.

Within the meaning of the invention, the expression "superior eukaryote" is intended to be understood to mean any eukaryote with the exception of any protist, namely, unicellular eukaryote. In a particular embodiment, superior eukaryote includes vertebrate and non-vertebrate animals, notably fishes, amphibians, reptiles, birds and mammals as well as gastropods, bivalves, insects, chelicerates, and crustaceans.

The inventors have also observed that it was possible to design a unique set of pairs of degenerate oligonucleotide primers to amplify a genomic DNA sequence of a gene of the histone H4 family, whatever the species to which such a gene belongs.

Within the meaning of the invention, the expression "genomic DNA sequence" is understood to mean all or part of any given DNA sequence coding or not for RNA and/or polypeptide molecules. It may be, for example, all or part of a gene, a promoter or any non-coding region of the genome.

The invention, according to one of its first aspects, is related to a method for amplifying a genomic DNA sequence, comprising at least steps of:
i) contacting said genomic DNA sequence with a set of pairs of degenerate oligonucleotide primers hybridizing with primers binding sites of said genomic DNA sequence, each of these sites being homologously present in at least two orthologous forms of said genomic DNA sequence,
ii) subjecting the mixture obtained at step i) to a touchdown polymerase chain reaction (PCR) under suitable conditions for obtaining said amplified DNA sequence.

Within the meaning of the invention, the expression "primer binding sites" is understood to mean a part of the nucleic acid sequence where the primer may hybridize even if some of the bases of this part of the nucleic acid sequence do not match to the corresponding base of the oligonucleotide primer.

Within the meaning of the invention, the terms "homologous" or "homologously" are understood to characterize a nucleic acid sequence of a gene which is significantly related to a nucleic acid sequence of another gene because of a close evolutionary relationship, either between species or within a species.

Within the meaning of the invention, the expression "orthologous genes" is understood to characterize genes in different species that are derived from the same gene in the last common ancestral species, and which usually have similar structure and functions.

According to another aspect, the instant invention is related to a method further comprising a hemi-nested touchdown PCR.

Within the meaning of the invention, the expression "hemi-nested PCR" is understood to mean a PCR which is carried out after a first PCR, with a pair or a set of pairs of oligonucleotide primers whose one member is nested between a pair or a set of pairs of primers used for the first PCR. And the second member is selected among the pair or the set of pairs of oligonucleotide primers used for the first PCR.

Within the meaning of the invention, the expression "nested primer" is understood to characterize a primer or a set of primers that hybridize at a distance away from and between the binding sites for a first pair or set of pairs of primers used in a first PCR.

Accordingly, in a hemi-nested PCR, as only one member of the pair or one group of the set of pairs of oligonucleotide primers is nested between the binding sites of the first pair or set of pairs of primers, the second member of the pair or second group of the set of pairs is binding to a site identical to one of the first pair or set of pairs of primers.

A nested PCR is a PCR in which the two members of the pair or the two groups of the set of pairs of oligonucleotide primers have binding sites located at a distance away from and between the binding sites of a first pair or a first set of pairs of oligonucleotide primers used in the first PCR.

According to another of its aspects, the instant invention has for object a method for identifying a species belonging to superior eukaryotes comprising at least the steps consisting in:
a) conducting a method according to the invention, and
b) characterizing an amplified DNA sequence issued from step a).

According to another of its aspects, the instant invention is also related to a degenerate oligonucleotide primer having nucleic acid sequence comprising from about 18 to about 30 bases pairs, and selected from nucleic acid sequences that hybridize from nucleotide 96 in 3'-5', or from nucleotide 307 in 5'-3', or from nucleotide 277 in 5'-3' of human HIST2H4 gene.

According to another of its aspects, the instant invention is also related to a degenerate oligonucleotide primer having nucleic acid sequence selected from SEQ ID NO 1, SEQ ID NO 2 or SEQ ID NO 3.

According to another of its aspects, the instant invention is also directed to the use of oligonucleotide primers according to the invention in the method of the invention for amplifying a genomic DNA sequence of a histone H4 gene.

According to another of its aspects, the invention is related to a kit for amplifying a genomic DNA sequence from a histone H4 gene comprising at least a set of pairs of degenerate oligonucleotide primers according to the invention.

The novel method of PCR is robust, highly sensitive and allows the assessing of the quality of genomic DNA extracted from samples taken from various animal species. Moreover, the method of PCR according to the invention may, also, be applied on DNA extracted from samples, the quality of which being highly variable.

The application of the method of PCR according to the invention allows the obtaining of a positive signal in at least 95 % of the tested samples.

Moreover, the sensitivity of the method according to the invention allows to detect a positive signal in sample containing very tiny amount of genomic DNA. It is possible to obtain a signal from sample containing genomic DNA in an amount ranging from about 0.1 fg to about 100 ng.

### DNA: possible source and extraction

The genomic DNA sequence onto which the method according to the invention may be applied may be a genomic DNA sample taken from animals belonging to non-vertebrate as well vertebrate branches.

Among vertebrates, that may be subjected to the method of the invention, mention may be made of fishes, amphibians, reptiles, birds and mammals.

As examples of non-vertebrate, that may be subjected to the method according to the invention, mention may be made of gastropods, bivalves, insects, chelicerates, and crustaceans.

The DNA samples may be extracted from various organic materials such as liver tissues, teeth, cell lines, or even faeces and saliva. The animal from which the DNA samples are extracted may be healthy or ill, such as for example suffering from an acute hepatitis or other various diseases that may lead to an alteration of the state of tissue to be sampled.

The method according to the invention may also be applied onto genomic DNA extracted from samples taken from living species or ancient and extinct species, such as, for example, living or dead gorillas and chimpanzees, as well as woolly mammoths *(Mammuthus primigenius).*

*The DNA may be extracted from samples according to any method known in the art by the skilled person (for example see Molecular Cloning: A Laboratory Manual (2*^{*nd*} *Ed.), Sambrook et al., 1989, Cold Spring Harbor Laboratory, Cold Spring Harbor Press, N.Y. (Sambrook)).* It is well known that, according to the nature of the biological material from which the DNA has to be extracted, the protocol to be applied must be adapted. For example, it is well known that conditions for extracting DNA from liver tissues, teeth, or fossilized species are different.

Protocol usable for extracting DNA from a sample taken from a tissue of a living species may comprise a digestion step in a proteinase containing buffers (e.g. SDS/proteinase K buffer), followed by an extraction step with organic solvent, for example, phenol and chloroform, and then a harvesting step of the DNA in an aqueous buffer after having precipited it from the organic phase by using solvent such as ethanol. The length of time, as well as the temperature, to which is conducted the digestion step may be routinely adjusted by the skilled person in the art. Typically, the temperature of digestion may vary from about 20 °C to about 40 °C, notably from 25 °C to about 37 °C, and the time of digestion may vary from about 4 to about 15 hours, in particular from 6 to about 12 hours.

For the extraction of DNA of faeces, the MASTERPURE TM complete DNA and RNA purification kit provided by EPICENTRE, may be, for example, used.

Regarding the DNA extraction from teeth and mammoth samples, it may be possible to refer to protocols previously described by Debruyne et al. (Mol. Phylogenet. Evol., 2003, 26:421-434) and Hassanin et al. (CR Acad. Sci. III, 1998, 321:611-620).

Whatever the protocol used to extract the DNA, it is, usually, required for PCR procedure, that the DNA be harvested in an aqueous buffer.

### Polymerase chain reaction

The polymerase chain reaction produces a large amount of a specific DNA sequence from a DNA template sequence in a simple enzymatic reaction. The method uses a DNA polymerase and two oligonucleotide primers to synthesize a specific DNA fragment from a template sequence.

The extracted genomic DNA containing the sequence to be amplified may be thereafter mixed with the other components of the PCR, namely, the oligonucleotide primers, the DNA polymerase and the four deoxynucleoside triphosphate, the overall being in a PCR buffer.

Usually, the PCR is performed in a total volume ranging from about 25 to about 100 µl.

Usually, 50 ng of genomic DNA may be subjected to the method according to the invention. However, the method according to the invention may efficiently produce an amplified genomic DNA sequence starting with an amount of DNA ranging from 0.1 fg to 100 ng. The starting amount of genomic DNA material may range from 20 fg to 1 ng, notably from 2 pg to 10 pg.

Within the meaning of the invention, the expression "amplified genomic DNA sequence" is understood to mean that multiple copies of a genomic DNA sequence or multiple copies complementary to the genomic DNA sequence have been constructed using at least one of the genomic DNA sequence as a template.

The DNA polymerase may usually be used in amount and conditions defined by the manufacturer. As example of DNA polymerase that may convene for implementing the method according to the invention, mention may be made of the Taq polymerase provided by LifeTechnologies. The concentration of DNA polymerase suitable for carrying out the method according to the invention may range, for example, from 0.05 to 0.2 unit/µl.

Usually, beside the provision of the enzyme, manufacturer of DNA polymerase usually provides the buffer in which the PCR is performed. A composition of a PCR buffer that may convene for the method according to the invention may comprise, for example, KCl (e.g. at about 50 mM), Tris (e.g. pH 8.9, at about 10 mM), Tween 20 (e.g. at about 0.1%), MgCl₂ (e.g. at about 1.5 mM) and dATP, dGTP, dTTP, dCTP (e.g. at about 200 µM each). However, the composition and concentrations of a PCR buffer may vary, depending on the DNA target sequence to be amplified, the DNA polymerase features or the oligonucleotide primers.

Each primer may be added to the buffer at a concentration ranging from about 0.5 to about 10 µM, and notably from about 0.5 to about 2 µM.

The above-discussed amount and composition of the PCR buffer, as well as the amount of oligonucleotide primers and DNA polymerase are given for, merely, illustrating the invention. And, it is of routine work for the skilled person in the art to adjust such amounts and composition according to the various possible uses of the invention.

The PCR is carried out in a series of cycles. Each cycle begins with a denaturation step to render the DNA target sequence single-stranded. This is followed by an annealing step during which the oligonucleotide primers anneal (or hybridize) to their complementary sequences so that their 3'hydroxyl ends face the target. Finally, each primer is extended trough the target region by the action of DNA polymerase, during an elongating step. These cycles are repeated over and over until a sufficient amount of amplified genomic DNA sequence is produced.

An usual PCR protocol may comprise a step of denaturation of the DNA sample, performed at about 90-96°C, for human genomic DNA, a step of annealing (or reannealing) at temperatures that may range from about 50°C to about 70°C, depending on the melting temperature of the expected duplex formed between the oligonucleotide primers and the target DNA sequence, and a DNA elongating step, carried out at a temperature ranging from about 70 to about 75°C.

The temperature of denaturation, annealing and elongating may be adapted according to the oligonucleotide primers, the nature of the DNA polymerase used for carrying out the synthesis, the ionic strength of the buffer and others well-known parameters to the skilled person in the art. For example, the widely used Taq DNA polymerase obtained from *Thermus aquaticus* is thermostable up to 94°C, with an optimum working temperature of 80°C. Therefore, the temperature of the synthesis step may be adjusted at a temperature ranging, for example, from about 68 to about 74°C and notably from about 70 to about 72°C.

The duration of each step, i.e. denaturating, annealing and elongating, is to be adapted according to the DNA target sequence to be amplified, the composition of the PCR buffer, the DNA polymerase features, the oligonucleotide primers, the concentration of each of those components. Usually, each step may last from about 15 seconds to about 5 minutes, from about 30 seconds to about 2 minutes or from about 45 seconds to about 1 minute.

Before engaging in the first cycle of denaturing, annealing and elongating, there may be a step, carried out one time, of denaturing performed at the same denaturing temperature that is used in the complete cycles of PCR, but that may last longer. Typically, such a step may last from about 3 to about 5 minutes, for example, it may last about 3 minutes and 15 seconds.

The PCR used in the method according to the invention is performed with an annealing temperature decreasing progressively as the PCR goes through the cycles. Such PCR is named "touchdown PCR" or "stepdown PCR".

As originally defined (Don et al., Nucleic Acid Res., 1991, 19:4008), the "touchdown PCR" is designed such that the annealing temperature of the first series of cycles, for example the first two cycles, is well above the estimated melting temperature of the duplex formed between the oligonucleotide primers and the target DNA sequence. Then, the annealing temperature of each subsequent series of cycles is run at 1°C less than the preceding series of cycles. Eventually, the melting temperature of the specific duplex will be reached and amplification will begin. By the time the temperature of the annealing cycles drops to the level where non specific primer would ordinarily occur, the specific product will have a geometric head start and should outcompete any non specific product for the remaining resources to yield a single predominant amplicon. The "stepdown PCR" is a variant of the "touchdown PCR", which is also well-known to the skilled person in the art and is, for example, described by Hecker and Roux (BioTechniques, 1996, 20:478-485). In that variant, the decrease of the annealing temperatures of the subsequent cycles may be performed with larger steps ranging from about 2°C to about 5°C.

Such protocols enhance the specificity of the PCR by avoiding mispriming during the initial cycles.

Within the rest of the description, the expression "touchdown PCR" will be used whatever is the stepwise decrease of the annealing temperature, i.e., 1 °C by 1°C, or larger step.

According to an embodiment, the touchdown PCR has an annealing temperature ranging from about 72 to about 45°C and notably from about 70 to about 50°C.

Schematically, a touchdown PCR may be described as follow:
α) conducting a cycle of PCR (comprising a step of denaturing, a step of annealing, a step of elongating),
β) repeating the cycle of step α) with a same annealing temperature,
γ) repeating step α) and step β) with a decrease of annealing temperature.

The steps α) to γ) are repeated in such a manner that the total number of cycles of the touchdown PCR is ranging from about 20 to about 60, from about 30 to about 50, from about 35 to about 45.

Particularly, the number of cycles of the touchdown PCR may be about 35.

In an embodiment of the invention, the touchdown PCR may be carried out with a stepwise decrease of annealing temperature ranging from about 1 to about 5°C, notably from about 2 to about 4°C, and in particular being of about 3°C.

According to another embodiment, the stepwise decrease of the annealing temperature along the last cycles of the PCR may be performed with steps larger than the steps used for the first cycles of the PCR. As for example, for decreasing the annealing temperature from about 72 or 70°C to about 64°C, it may be possible to use steps of 3°C, then for decreasing the annealing temperature from about 64°C to about 50 or 45°C, it may be possible to use steps of 5°C.

According to another embodiment, the touchdown PCR carried out may be followed by a hemi-nested touchdown PCR.

For the hemi-nested touchdown PCR, the amplified DNA sequence issued from the touchdown PCR is contacted with a set of pairs of degenerate oligonucleotide primers hybridizing with primer binding sites of said amplified DNA sequence, wherein at least one of said sites is nested between the primer binding sites of the primers used in the touchdown PCR, and each of those sites is present in, at least 2 orthologous forms of the genomic DNA sequence that has been subjected to the touchdown PCR.

Then the touchdown protocol of the hemi-nested PCR is carried out as previously described, in suitable conditions for further amplifying the amplified DNA sequence.

The further amplified DNA sequence obtained after the hemi-nested touchdown PCR is shorter than the amplified DNA sequence obtained after the touchdown PCR.

In order to improve the signal to noise ratio, it may be possible to add to the mixture subjected to the second PCR a solvent allowing for background level reduction. Numerous solvents are known in the field to be suitable in PCR to increase the specificity and the signal relative to the background noise.

As example of solvent suitable for carrying out the method according to the invention, mention may be made of DMSO, formamide or betaine.

When used, the DMSO may be advantageously present in the mixture subjected to the second PCR in a proportion ranging from 4 to 10% in volume relative to the total volume of the mixture, notably from 6 to 8% relative to the total volume of the mixture.

According to another embodiment, the DMSO may be present in the mixture in a proportion of 7% by volume relative to the total volume of the mixture.

According to another embodiment, the mixture or a fraction of the mixture obtained after the touchdown PCR may be diluted before being subjected to the hemi-nested touchdown PCR.

The dilution may be carried out in a ratio of 1:10 to 1:25 of the volume of the first PCR.

According to another embodiment, the genomic DNA sequence to be amplified, by the method, may be of at least 120, notably of at least 150, notably of at least 180, notably of at least 200, and notably of at least 210 base pairs.

According to another embodiment, the sets of pairs of degenerate oligonucleotide primers to be used in the method, hybridizes to primers binding sites of the genomic DNA sequence homologously present in at least 5, notably in at least 10, notably with at least 20, notably in at least 40, notably in at least 60, and notably in at least 80 orthologous forms of said genomic DNA sequence.

According to an embodiment, genomic DNA sequence to be amplified is all or part of a gene of the histone family. In particular, this gene may be a gene from the histone H4 family. More particularly, this gene may be HIST2H4, and notably the histone H4 gene subjected to the method of the invention may be the human HIST2H4 gene.

### Characterisation of the amplified DNA sequence

According to another embodiment, further to the amplification of the genomic DNA sequence as above-described, the said amplified DNA sequence may be subjected to a step of characterization.

The additional step of characterization may allow the implementing of a method for identifying species belonging to superior eukaryotes. The characterization of the amplified DNA sequence may allow its comparison with others DNA sequences, stored in at least databases.

According to an embodiment, when the amplified DNA sequence is from a gene of the histone H4 gene family, it may be compared with DNA sequences of orthologous histone H4 genes stored in databases.

According to another embodiment, the step of characterization may be carried out by sequencing the amplified genomic DNA sequence, and/or testing for the presence of one or more restriction sites, and/or probing the amplified DNA sequence with a specific DNA or RNA probe.

The step of characterization of the amplified genomic DNA sequence is advantageously performed after purification of the amplified fragment.

Such purification may be performed by any known method in the field such as magnetic beads or on hydroxylated silica columns or according to the phenol chloroform method, followed by a precipitation step.

The step of sequencing may be carried out after ligating the amplified genomic DNA sequence into a cloning vector and then sequencing it by using an automated system.

The cloning vector to be used is, advantageously, of the A-overhang type, well-known by the skilled person in the art.

Routinely, to assure a good certainty with respect to the results of the sequencing, this latter may be carried out on various clones, for example, from about 3 to about 5 clones.

As example of cloning vector suitable for carrying out the instant invention, mention may be made of the TOPO TA® cloning vector provided by INVITROGEN.

As example of automated system for sequencing that may convene for performing the instant invention, mention may be made of ABI 373A SEQUENCER provided by APPLIED BIOSYSTEMS, using Thermosequenase kit provided by USB.

When the sequence of the amplified genomic DNA sequence is obtained, this sequence may be aligned by using various softwares and then compared with other DNA sequences stored in various databases. Various software and methods are known by the skilled person in the art to align and compare DNA sequences. As example, mention may be made of the method described by Higgins *et al.,* Nucleic Acid Res., 1994, 22:4673-80.

The characterization of the amplified genomic DNA sequence by identification of restriction sites may rely upon the digestion of the amplified genomic DNA sequence with various restriction enzymes. Thereafter, the gel shift migration of the various fragments resulting from the digestion may be observed and analysed. The amplified genomic sequence may be identified according to the presence or absence of various restriction sites.

The amplified genomic DNA sequence of each orthologous histone H4 gene may display a particular profile of fragments resulting from the digestion with restriction enzymes allowing its subsequent characterization.

Another suitable means to characterize the sequence of the amplified genomic DNA sequence is to probe the sequence with specific DNA or RNA probe (e.g. fluorescent) which are complementary and hybridizes to the amplified genomic DNA sequence, for example under high-stringency conditions.

### Degenerate oligonucleotide primers

Other objects of the instant invention are sets of pairs of degenerate oligonucleotide primers to be used in the method according to the invention.

A set of pairs of degenerate oligonucleotide primers may be obtained for any gene, provided that such gene exists at least under the form of 2 orthologous genes into animal species. Consequently, the method according to the invention may be applied, virtually, to any gene, provided that orthologous forms of this gene exist. Sets of pairs of degenerate oligonucleotide primers may be obtained by performing an alignment of known sequences of orthologous genes.

The primers are named degenerate owing to the fact that the genetic code is degenerate. The genetic code is a three-letter code. There are four possible bases to choose from each of the three base positions in a codon.

Consequently, there are 64 possible codons, but only 20 different aminoacids are specified. As a result, the genetic code is said to be degenerate: each aminoacid is specified on average by about 3 different codons. Consequently, even if the sequence of a given protein is highly conserved among various animal species, the corresponding nucleic acid sequence encoding for such protein may display a highest variability owing to the degeneration of the nucleic acid code.

Therefore, when designing set of degenerate oligonucleotide primers according to the invention, one should take into account the degeneration of the nucleic acid code. Consequently, in a sequence of a given degenerate oligonucleotide primer may exist several possibility of base. A degenerate oligonucleotide primer encompasses one or more possibilities. Therefore a degenerate oligonucleotide primer or a set of degenerate oligonucleotide primer of a given sequence is a set of primers corresponding to those possibilities.

Other variables must be taken into account when designing PCR primers. Among the most critical are: primer length; melting temperature (Tₘ); specificity; complementary primer sequences; G/C content and polypyrimidine (T,C) or polypurine (A,G) stretches 3'-and sequence.

Specificity, temperature, and time of annealing are at least partly dependent on primer length. Therefore, this parameter is critical for successful PCR. In general, oligonucleotides between 18 and 30 bases are extremely sequence specific.

Therefore, the length of the degenerate oligonucleotide primers of the invention may be, advantageously, in the range of about 18 to about 30 nucleotides, in particular of about 25 nucleotides, more particularly of about 26 nucleotides.

The annealing efficiency is depending on the primer length. Typically, the goal should be to design an oligonucleotide primer with an annealing temperature of at least 50°C.

Numerous methods, known from the skilled person in the art, may be used to calculate the melting temperature of the oligonucleotide primers (see for example, Breslawer et al., Proc.Natl. Acad. Sci. USA, 1986, 83:3746-50; Freier et al., Proc.Natl. Acad. Sci. USA, 1986, 83:9373-9377; and Schildkraut et al., Biopolymers, 1965, 3:195-208.)

Moreover, the set of pairs of degenerate oligonucleotide primers that may be suitable to carry out the method according to the invention should allow to amplify a genomic DNA sequence or an amplified genomic DNA sequence of at least 120, notably of at least 150, notably of at least 180 and notably of at least 200 base pairs.

As example, a length of genomic DNA sequence to be amplified according to the touchdown PCR may be of 211 base pairs.

A set of pairs of degenerated primers may be designed to be implemented into a hemi-nested touchdown PCR as previously described. That is, advantageously, one member of the pairs may hybridize with a primer binding site of the amplified genomic DNA sequence which is nested between the primer binding sites of the set of pairs of degenerate oligonucleotide primers used for the touchdown PCR.

In a particular embodiment, the set of pairs of degenerate oligonucleotide primers that may be useful for the hemi-nested touchdown PCR, as previously described, may allow the amplification of a DNA sequence having a nucleic acid sequence length of 181 base pairs.

The set of pairs of degenerate oligonucleotide primers that may convene to carry out the method of the invention may hybridize to primer binding sites of a genomic DNA sequence which are homologous present in at least 2, in at least 5, in at least 10, in at least 20, in at least 40, in at least 60 and in at least 80 orthologous genes.

The inventors have selected a gene coding for a histone protein owing to its highly conservation among species to design sets of degenerate oligonucleotide primers.

The synthesis of the sets of pairs of primers of the invention may be carried out by any known method in the field by the skilled person.

In a particular embodiment, the histone gene to be used in the method according to the invention and to be used as template to design the sets of pairs of degenerate primers is a histone H4 gene, and more particularly, it is the HIST2H4 gene.

According to a particular embodiment, the degenerate oligonucleotide primers may be designed to hybridize with conserved regions of the human HIST2H4 gene which are homologously present in corresponding orthologous genes.

According to another embodiment, the degenerate oligonucleotide primers may be designed to have a nucleic acid sequence comprising from about 18 to about 30 base pairs, and selected from nucleic acid sequences that hybridize from nucleotide 96 in 3'-5' or from nucleotide 307 in 5'-3' or from nucleotide 277 in 5'-3', of human HIST2H4 gene.

The set of pairs of degenerated primers, suitable for the touchdown PCR as previously described, may contain primers having nucleic acid sequence selected from:
- H4F2s, SEQ ID NO 1: 5'TSCGIGAYAACATYCAGGGIATCAC, and
- H4F2er, SEQ ID NO 2: 5'CKYTTIAGIGCRTAIACCACRTCCAT,
wherein I is deoxyinosine; K is deoxyguanosine or deoxythymidine (G or T); R is deoxiadenosine or deoxyguanosine (A or G); S is deoxycytidine or deoxyguanosine (C or G); and Y is deoxycytidine or deoxythymidine (C or T).

In the above-described primer sequences, the deoxyribonucleic acids are written in a one-letter code. In this code, C is deoxycytidine, T is deoxythymidine, A is deoxyadenosine, and G is deoxyguanosine.

The set of pairs of degenerated primers that may convene to implement the hemi-nested touchdown PCR may comprise primers having nucleic acid sequences selected from:
- H4F2s, SEQ ID NO 1: 5'TSCGIGAYAACATYCAGGGIATCAC, and
- H4F2ir, SEQ ID NO 3: 5'GTIACIGTCTTSCKYTTGGCGTGCTC.

The above oligonucleotide primers are also written in a one-letter code whose meaning is as defined for the first set of pairs of degenerate oligonucleotide primers.

As discussed above, the touchdown PCR carried out in the method of the invention may be, or not, followed by a hemi-nested touchdown PCR. When the first touchdown PCR is not followed by the hemi-nested touchdown PCR, it may be envisioned to use as set of pairs of degenerate primers the set provided by the combination of primers of SEQ ID NO 1 and primers of SEQ ID NO 3.

Owing to the degeneration of the nucleic acid code as above-described, it is understood that under the primer name SEQ ID NO 1 (as well as for the NO 2 and 3) there are in fact numerous sequences of primers. The instant invention also relates to each of those primers.

According to another embodiment, the instant invention is also directed to oligonucleotide degenerate primers having from 18 to 30 bases comprising at least 18 contiguous bases selected from SEQ ID NO 1, SEQ ID NO 2 or SEQ ID NO 3 to up the full sequence of SEQ ID NO 1, SEQ ID NO 2 or SEQ ID NO 3.

The instant invention is also directed to a kit for amplifying a genomic DNA sequence from a histone gene, in particular a histone H4 gene, and more particularly the HIST2H4 gene, comprising at least a set of pairs of degenerate oligonucleotide primers as previously defined.

According to another embodiment, the kit may also comprise the reagents required for carrying out PCR as well as the reagents for characterizing an amplified genomic DNA sequence.

The method according to the invention may be applied in numerous field, such as the control quality of a genomic DNA from any animal species belonging as well as to vertebrate than to invertebrate branches. The method may also be applied into forensic entomology for the molecular characterization of larvae of dipters in order to assess the post-mortem interval.

During police investigations of biological remains, the human origin or the animal species origin may be easily attributed to any biological remains by using the method of the invention.

An advantage of the use of the method according to the invention in the forensic entomology is a fast identification and a high degree of certainty that may be provided. Using the method in accordance with the invention on DNA extracted from larvae taken from corpses allows a fast identification of the larvae without requiring the culture of the larvae to allow their transformation into adult insects, and the subsequent identification of those insects.

Another possible application of the method of the invention is the identification of the species of an animal having bitten someone and thereafter having disappeared. By using the saliva taken from the wound and applying to such sample the method according to the invention, it may possible to identify the animal responsible for the wounding.

It is obvious to the skilled person in the art that it may be possible to bring numerous modifications and variations to the above-exposed invention without departing from the scope of the invention. It is intended to also over such modifications and variations.

The invention will be more fully understood by reference to the following examples. They should not, however, be construed as limiting the scope of the invention.

### LEGENDS OF THE FIGURES AND THE TABLES

Figure 1: It shows the amplification of HIST2H4 from partially degraded DNA Samples.
Fig. 1A: Partially degraded DNAs were controlled on a 0.8% agarose gel. Seven DNA samples were extracted from necropsy specimens, (lanes 1-7, *Agama agama, Lamprophis fuliginosus, Mus domesticus, Gallus gallus, Roussettus aegyptiacus, Fennecus erda, Suricatta suricatta);* three poor quality DNA samples from non vertebrates (Lane 8-10, *Spodoptera frugiperda, Arion hortensis, Attya mollucensis);* lane 11 contains the positive control (COS7 cells from *Cercopithecus aethiops).*
Fig. 1B: The 181bp amplification products obtained after HIST2H4 hemi-nested PCR performed on each of the degraded DNAs samples were analysed on an agarose ge1.

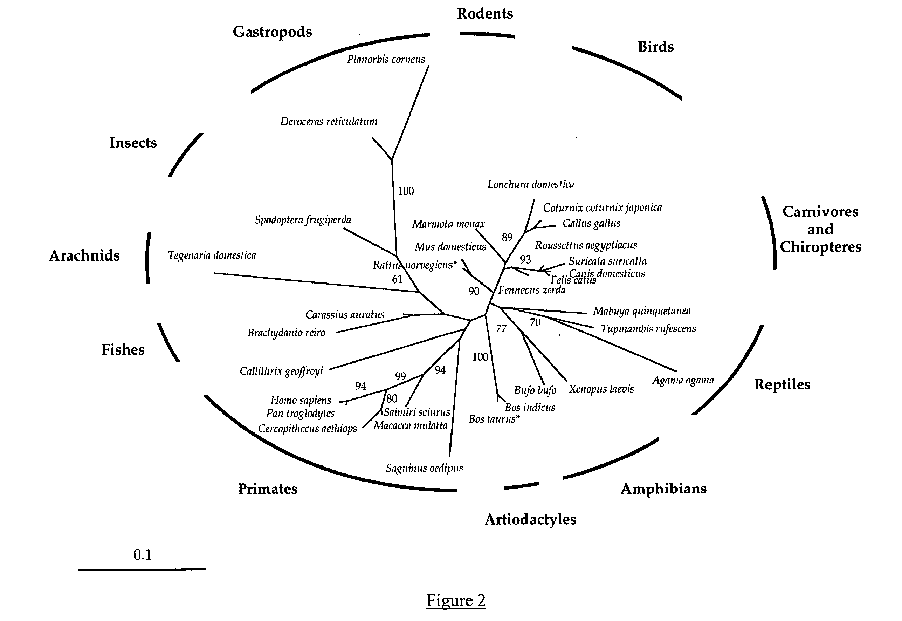

Figure 2: It shows an unrooted tree of HIST2H4 genes. The twenty-nine most representative HIST2H4 sequences were aligned and used to construct the tree (NJ, Kimura2-parameter). Bootstrap values are based on 100 replications and only those above 60% are shown. Asterisks represent sequences taken from the gene bank, respectively *Rattus norvegicus* (accession, NM 022686) and *Bos Taurus* (accession, NM 173880).

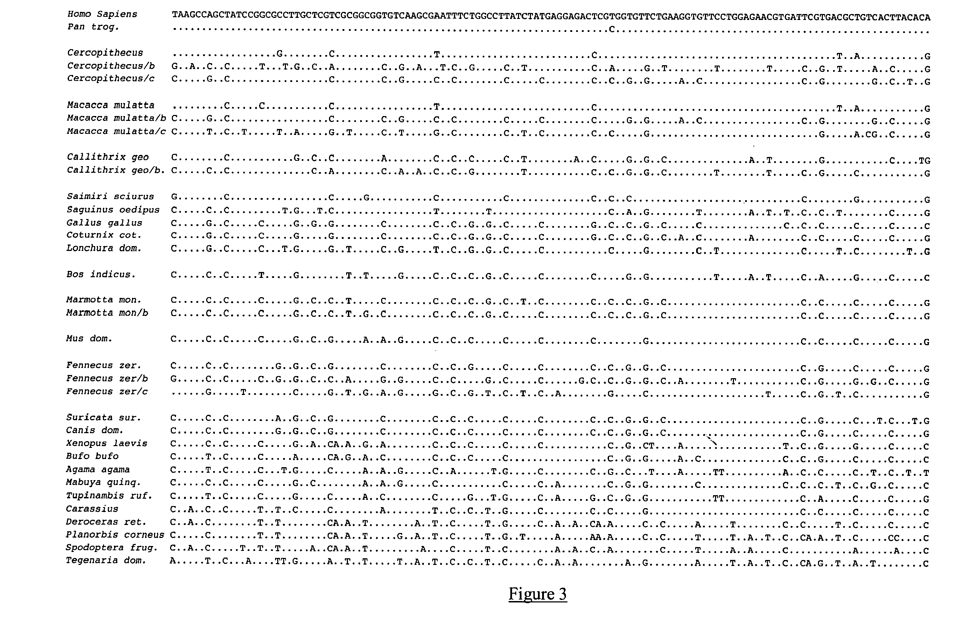

Fig. 3: Alignment of the 181 bp sequence amplified from HIST2H4 from the tested samples.

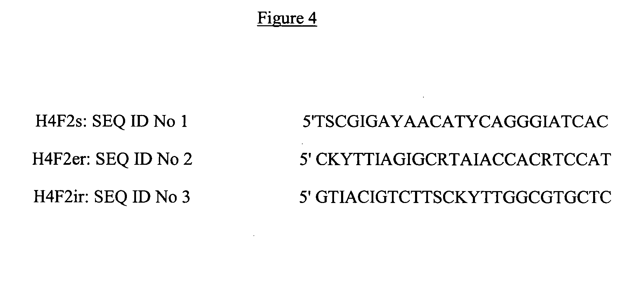

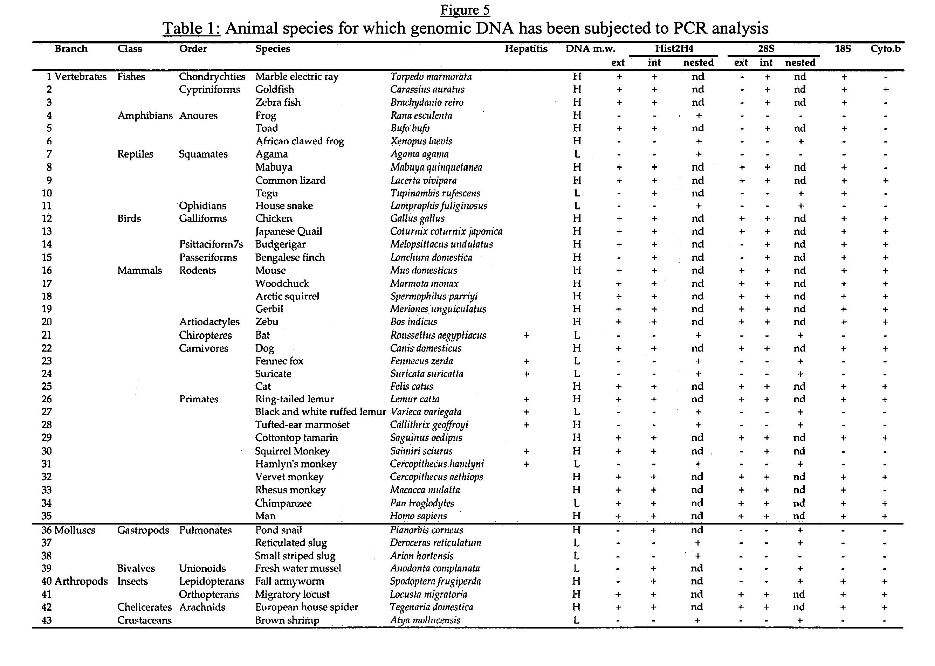

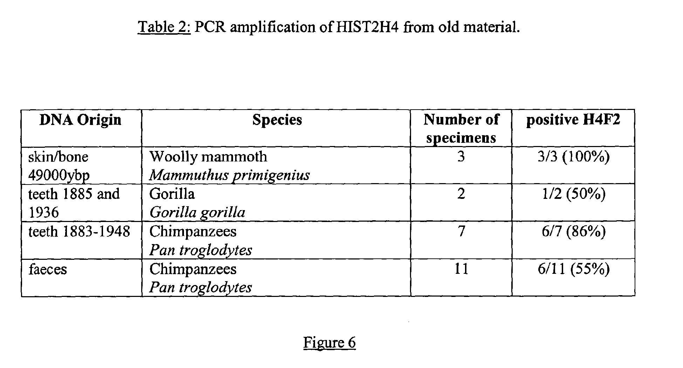

Fig. 4: Sequences of the degenerate oligonucleotide primers.

Table 1: It lists the animal species for which genomic DNA has been subjected to PCR analysis. Taxonomic data as branches, class, order, English vernacular names and zoological names are mentioned in the 5 leftmost columns. Hepatitis indicates whether it was a necropsy sample from animal that died from hepatitis. DNA molecular weight indicates the presence of high (H) or low (L) molecular weight DNA fragments. In the following columns are reported the PCR data from HIST2H4 and 28S rDNA nested PCR as well as 18S rDNA and cytochrome b after a single round of PCR. + an - indicate a positive or negative PCR results respectively. M.w: molecular weight, nd: PCRs have not been performed. Ext, int, and rested respectively indicate PCR carried-out with a first set of primers, a second set of primers, and a PCR carried-out with the first set of primers followed by a PCR carried-out with the second set of primers (hemi-nested).

Table2: It lists the PCR amplification of HIST2H4 carried out on ancient material. All PCRs displayed in this table were performed according to the hemi-nested procedure.

### MATERIAL AND METHODS

### Samples

Tissues samples from necropsies and from cell lines belonging to 43 different species were studied. Those samples including 8 samples from invertebrates and 35 from vertebrates are listed in Table 1.

Among vertebrates, all traditional classes (fishes, amphibians, reptiles, birds, mammals) were represented. A subset of DNAs studied (n=8) were extracted from liver tissues obtained from animals suffering from acute hepatitis whereas remaining samples were obtained from established cell lines (e.g. COS7 from Cercopithecus aethiops), laboratory model specimens (e.g. zebra fish or chicken), routine practice in veterinary medicine (ovarian or testicular ablation from dog and cat respectively). In addition, DNA was extracted from three groups of different samples, ancient and extinct species, 11 faeces from chimpanzees collected in the wild, 9 teeth of gorilla and chimpanzees collected between 1883 and 1948 and finally 3 samples from Siberian woolly mammoths *(Mammuthus primigenius)* conserved in permafrost since the Pleistocene (Debruyne et al., Mol. Phylogenet. Evol., 2003, 46:344-354).

### DNA extraction

Samples from necropsies and from cell lines were digested in SDS/proteinase K buffer (0.1 mg/ml) at 37°C for 6 to 12 h, and extracted twice with phenol and once with chloroform. DNA was then ethanol-precipitated and resuspended in TE buffer. Besides liver necropsies, a collection of samples considered as degraded was investigated.

Faeces from chimpanzees living in the wild were extracted using the MasterPure TM® complete DNA and RNA purification kit (Epicentre® , Tebu-bio, Le Perray-en-Y., France) according to the manufacturer's procedure. Nine DNA samples were extracted from teeth of chimpanzees (n=7) and gorillas (n=2) dating from between 1883 and 1948.

DNA samples from siberian woolly mammoths *(Mammuthus primegenius)* and from tooth and mammoth DNA were prepared according to protocols previously described (Debruyne et al., Mol. Phylogenet. Evol., 2003, 46:344-354; Hassanin et al., CR Acad. Sci. III, 1998, 321:611-620).

### Design of the primers

Degenerate primers were designed in a conserved region of the human HIST2H4 gene (1q21) and its orthologous published in databases.

For the first round of amplification, the amplified segments were mapped between nucleotides 96 and 307 (211 bp) of HIST2H4 gene (accession # BC019846): H4F2s, SEQ ID NO 1 5'TSCGIGAYAACATYCAGGGIATCAC and H4F2er, SEQ ID NO 2 5' CKYTTIAGIGCRTAIACCACRTCCAT, (I: inosine, K: G/T; R:A/G; S: C/G; Y: C/T). For the second round of amplification, the primers were mapped between nucleotides 96 and 277 (181 bp) of HIST2H4 gene: H4F2er et H4F2ir SEQ ID NO 3 5' GTIACIGTCTTSCKYTTGGCGTGCTC.

### Polymerase chain reaction

Approximately, 50 ng of genomic DNA were subjected to 35 cycles of amplification according to a "stepdown" protocol in a Techne Genius® thermal cycler (OSI, Saint-Quentin-en-Yvelines, France) (Hecker and Roux, Biotechniques, 1996, 20:478-485) in a first round of PCR. Reactions were performed in 25 µl of a mixture containing 1X PCR buffer [50 mM KCl, 10 mM Tris (pH 8.9), 0.1 % Tween 20, 1.5 mM MgCl2, 200 µM dATP, dGTP, dTTP, dCTP], 50 pmol of each set of primers, 1.25 unit of Taq polymerase (Life Technologieses® , Cergy-Pontoise, France). For the first round of PCR, H4F2s and H4F2er were used.

For the hemi-nested protocol, primer H4F2s was combined with H4F2ir on 2 µl of the primary amplification and subjected to 35 additional PCR cycles according to the same procedure except that reactions were performed in the presence of 7% DMSO. Primers used to amplify 28S rRNA, 18S rRNA and cytochrome b PCR have been published elsewhere (Kocher et al., Proc. Natl. Acad. Sci. USA, 1989, 86:6196-6200; Meyer et al., I Acad. Int., 1994, 77 :617-622; Naito et al. , J. Forensic Sci., 1992, 37 :396-403).

Following PCR, amplification products were loaded on a 2% agarose gel (Eurobio® , Les Ulys, France), stained with ethidium bromide and visualized on a UV transilluminator. Appropriate precautions to avoid cross contamination were taken while performing the PCR and negative amplification controls remained negative.

### Sequences analysis

For all 43 species amplified fragments were purified from agarose gels (Qiaex II kit® , Qiagen® , France) and ligated in the TOPO TA® cloning vector (InVitrogen® , France). Sequencing was performed using Thermosequenase (USB® , Amersham) on an ABI373A sequencer® (Applied Biosystems® ). Five clones were sequenced for each of these 43 species. In addition, between 3 and 5 clones from each mammoth specimens were sequenced. Sequences were aligned by using the CLUSTAL W program (Thompson et al., Nucleic Acid Res., 1994, 22:4673-4680). Nucleotide distances were determined with DNAdist of the Phylip package version 3.5 (Felsenstein, 1993, Phylip, version 3.5, distributed by the author Deartment of Genetics, University of Washington, Seattle, USA). The tree was derived by neighbor-joining (NJ) analysis applied to pairwise distances calculated using the Kimura two-parameter method to generate unrooted trees. Robustness of the NJ phylogenetic tree was tested by boostrap analysis. Horizontal branch lengths are drawn to scale with the bar indicating 0.1 nucleotides replacements per site. The final output was generated with Treeview (Page, Computer Applications in the Biosciences, 1996, 12:357-358). The number at each node represents the percentages of bootstrap replicates (out of 100). Only bootstrap values of > 60 are given. The sequences can be found in GenBank under accession numbers: AY675260-AY675296.

### Example I

### Amplification from vertebrates, non-vertebrates and clinical samples

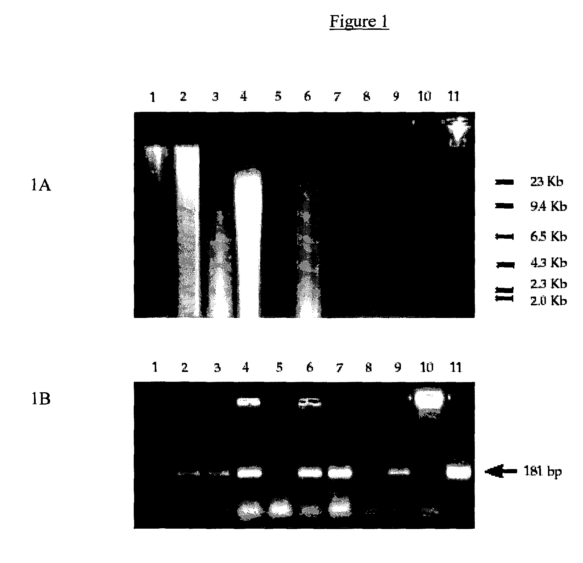

All vertebrate samples (n=35) yielded amplification products either after a single round or a hemi-nested amplification procedure (Table 1). Those of the vertebrates samples yielding amplimers only after the second round of amplification originated in most cases (9/11 cases) from degraded tissues specimens obtained from animals suffering from severe acute hepatitis or from inappropriately stored samples (Figure 1A and 1B). In the remaining cases (n=25/36, 67%), a single round of PCR cycles was sufficient to obtain a detectable amplimer. As expected, after 35 cycles, amplification of the smaller 6 internal (181 bp) fragment was overall more efficient than that of the larger one (211 bp, 25 vs. 20 positive samples). For invertebrates, the hemi-nested procedure was often necessary (n=3/9) to obtain a sufficient amplification product (Table 1).

### Example II

### Comparison with previously published wide-spectrum PCR methods

To ascertain the usefulness of the method, the performance of HIST2H4 amplification was compared with those of previously published universal PCR on the same DNA samples.

18S/28S rRNA or cytochrome b amplifications were reviewed.

The performance of HIST2H4 amplification was similar to that of 28S rRNA amplification, with 43 (95%) and 41 (91%) positive samples, respectively. Both techniques were performed according to a nested and hemi-nested procedure, thus explaining the high fraction of successful assays.

The performances of 18S rRNA and cytochrome b PCRs were poorer, with 25 (55%) and 17 (38%) positive samples, respectively (Table 1). This difference is most likely attributable to the lack of a nested procedure.

The sequenced DNA from HIST2H4 gene were occupying in the phylogenic tree, a position consistent with their place in evolution indicating the robustness of the tree. However, with the primers used in the current report amplification precludes the detection of HIST1H4 B, C, D, E, G and HIST4H4, out of the 14 human histone H4 genes due to the presence of disparate mismatches in the third base of the primer sequences. Hence, the alignment of HIST2H4 sequences gave a major form of 80% (4/5), however, in some animal species, 1 or 2 HISTH4 sequences were also amplified from another histone H4 locus.

### Example III

### Relative sensitivity of the method

The amount of DNA required for HIST2H4 assay was next examined. An absolute comparison on different genomes is not possible because the exact genome sizes and composition with regard to the number of histone H4 genes are not known for most of the animal species. Broad differences in amplification efficiency attributable to the number of histone H4 genes per genome (sometimes in accordance with genome size) or to particular homology with degenerate primers may be detected by comparing different high molecular weight DNA samples (Bensasson et al., Mol. Biol. Evol., 2001, 18:246-53; Ishaq et al., Biochem. Biophys. Res. Comm., 1993, 194:775-783).

Six different samples were tested by serial ten-fold dilutions ranging from 100 ng to 0.1 fg. Species were chosen in each of the vertebrate classes *(Bufo bufo, Mabuya quinquetanea, Melopsittacus undulatus and Cercopithecus aethiops)* and a non-vertebrate was also added *(Locusta migratoria).* First round of amplification yielded clear amplimeres within a range of 1 ng *(Bufo bufo, Mabuya quinquetanea, Melopsittacus undulatus, Cercopithecus aethiops)* to 10 pg *(Locusta migratoria)* whereas the second round of PCR extended the detection range from 2 pg *(Mabuya quinquetanea, Cercopithecus aethiops)* to 20 fg *(Locusta migratoria, Bufo bufo, Melopsittacus undulatus).*

### Example IV

### Degraded, ancient and extinct DNA

Some samples, considered as difficult to explore i.e. chimpanzee faeces, ape teeth and mammoth tissues were then subjected to PCR for the HIST2H4 target gene. Amplification was successful in 6 out of 11 DNA samples extracted from chimpanzee faeces (55%) whereas ape teeth yielded a product in 7 out of 9 cases (77%). The oldest DNA extracted from a tooth was dating plify 3/3 Siberian mammoth samples dating from 40,000 to 49,000 years before present (Table 2). These data indicate that HIST2H4 amplification is robust enough to be performed on modem and old remains.

## Claims

1. A method for amplifying a genomic DNA sequence, comprising at least steps of:
iii) contacting said genomic DNA sequence with a set of pairs of degenerate oligonucleotide primers hybridizing with primers binding sites of said genomic DNA sequence, each of these sites being homologously present in at least two orthologous forms of said genomic DNA sequence,
iv) subjecting the mixture obtained at step i) to a touchdown polymerase chain reaction (PCR) under suitable conditions for obtaining said amplified DNA sequence.

2. The method according to the preceding claim, further comprising a step of:
v) subjecting the amplified DNA sequence obtained according to claim 1 to a hemi-nested touchdown PCR under suitable conditions for further amplifying said amplified DNA sequence.

3. The method according to anyone of the preceding claims, wherein the genomic DNA sequence to be amplified is of at least 120, notably of at least 150, notably of at least 180, notably of at least 200, and notably of at least 210 base pairs.

4. The method according to anyone of the preceding claims, wherein said primer binding sites are homologously present notably in at least 5, notably in at least 10, notably in at least 20, notably in at least 40, notably in at least 60, and notably in at least 80 orthologous forms of said genomic DNA sequence.

5. The method according to anyone of the preceding claims, wherein the set of pairs of degenerate oligonucleotide primers comprises primers having a nucleic acid sequence length ranging from 18 to 30 nucleotides, notably of at least 25 nucleotides, and notably of at least 26 nucleotides.

6. The method according to anyone of the preceding claims, wherein the genomic DNA sequence is all or part of a gene of histone family.

7. The method according to the preceding claim, wherein the gene is a gene from the histone H4 family.

8. The method according to the preceding claim, wherein the gene is HIST2H4.

9. The method according to anyone of the preceding claims, wherein the set of pairs of degenerate oligonucleotide primers of claim 1 comprises primers having nucleic acid sequences selected from SEQ ID NO 1 and SEQ ID NO 2.

10. The method according to anyone of claims 2 to 9, wherein the hemi-nested touchdown PCR is carried out with a set of pairs of degenerate oligonucleotide primers comprising primers having nucleic acid sequences selected from SEQ ID NO 1 and SEQ ID NO 3.

11. The method according to anyone of the preceding claims, wherein the PCR of claim 1 and/or the PCR of claim 2 have an annealing temperature ranging from 72 to 45°C, notably from 70 to 50°C.

12. The method according to anyone of the preceding claims, wherein the PCR of claim 1 and/or the PCR of claim 2 comprise a number of cycles ranging from 20 to 60, from 30 to 50, from 35 to 45.

13. The method according to anyone of the preceding claims, wherein the PCR of claim 1 and/or the PCR of claim 2 are performed with a stepwise decrease of an annealing temperature ranging from 1 to 5°C, notably from 2 to 4°C, and notably is of 3°C.

14. The method according to claim 13, wherein the PCR of claim 2 is carried-out on or mixture comprising additionally a solvent selected form DMSO, formamide or betaine.

15. The method according to anyone of claims 2 to 14, wherein the amplified DNA sequence is diluted before being subjected to the hemi-nested touchdown PCR.

16. A method for identifying a species belonging to superior eukaryotes comprising at least the step consisting in:
a) conducting the method defined according to anyone of claims 1 to 15, and
b) characterizing an amplified DNA sequence issued from step a).

17. Degenerate oligonucleotide primer having a nucleic acid sequences comprising from 18 to 30 bases pairs, and selected from nucleic acid sequences that hybridize from nucleotide 96 in 3'-5', or from nucleotide 307 in 5'-3', or from nucleotide 277 in 5'-3' of human HIST2H4 gene.

18. Degenerate oligonucleotide primer having a nucleic acid sequence selected from SEQ ID NO 1, SEQ ID NO 2 or SEQ ID NO 3.

19. An oligonucleotide primer selected from the degenerate oligonucleotide primers as defined according to claim 17 or 18.

20. A use of oligonucleotide primer defined according to anyone of claims 17 to 19, in a method for amplifying a genomic DNA sequence of a histone H4 gene.

21. The use according to the preceding claim, wherein the method is as defined according to anyone of claims 1 to 15.

22. A kit for amplifying a genomic DNA sequence from a histone H4 gene comprising at least a set of pairs of degenerate oligonucleotide primers as defined according to anyone of claims 17 to 19.


SEQUENCE LISTING

<110> INSTITUT NATIONAL DE LA SANTE ET DE LA RECHERCHE MEDICALE (INSERM)
INSTITUT PASTEUR

<120> Method for universal PCR

<130> BR39187/CR/HG/klp

<140> 04 292994.3
<141> 2004-12-14

<160> 3

<170> PatentIn version 3.1

<210> 1
<211> 25
<212> PRT
<213> Homo Sapiens

<400> 1

Thr Ser Cys Gly Ile Gly Ala Tyr Ala Ala Cys Ala Thr Tyr Cys Ala
1               5                   10                  15

Gly Gly Gly Ile Ala Thr Cys Ala Cys
            20                  25

<210> 2
<211> 26
<212> PRT
<213> Homo Sapiens

<400> 2

Cys Lys Tyr Thr Thr Ile Ala Gly Ile Gly Cys Arg Thr Ala Ile Ala
1               5                   10                  15

Cys Cys Ala Cys Arg Thr Cys Cys Ala Thr
            20                  25

<210>  3

<211>  26

<212>  PRT

<213>  Homo Sapiens

<400>  3

Gly Thr Ile Ala Cys Ile Gly Thr Cys Thr Thr Ser Cys Lys Tyr Thr
1               5                   10                  15

Thr Gly Gly Cys Gly Thr Gly Cys Thr Cys
            20                  25